# EUROPÄISCHE PATENTANMELDUNG

(11) **EP 1 649 819 A1**
(43) Veröffentlichungstag der Anmeldung: **26.04.2006**
(21) Anmeldenummer: 04029379.7
(22) Anmeldetag: 10.12.2004
(51) Int. Cl.: A61B 17/80

(54) **Knochenplatte**

(30) Priorität: 19.10.2004 AT 75704 U
(71) Anmelder: Maier, Christian, 8280 Fürstenfeld (AT)
(72) Erfinder: Maier, Christian, 8280 Fürstenfeld (AT)
(74) Vertreter: Torggler, Paul Norbert

(57) **Zusammenfassung**

Knochenplatte (1) mit wenigstens einer Durchtrittsöffnung (2) für eine Knochenschraube (3), wobei in der Durchtrittsöffnung (2) verformbare Mittel (4) zum winkelstabilen Festlegen einer Knochenschraube (3) unter verschiedenen Winkeln (a) angeordnet sind, wobei die Mittel (4) zum Festlegen der Knochenschraube (3) wenigstens zwei, vorzugsweise vier, in einer zur Knochenplatte (1) im Wesentlichen parallelen Ebene (E) angeordnete Vorsprünge umfassen.

## Beschreibung

Die Erfindung betrifft eine Knochenplatte mit wenigstens einer Durchtrittsöffnung für eine Knochenschraube, wobei in der Durchtrittsöffnung verformbare Mittel zum winkelstabilen Festlegen einer Knochenschraube unter verschiedenen Winkeln angeordnet sind.

Bei den bisher bekannten derartigen Knochenplatten sind in den Durchtrittsöffnungen für die Knochenschraube umformbare Mittel zum Festlegen der Knochenschraube unterhalb konischer oder sphärischer Sitzflächen für den Kopf der Knochenschraube ausgebildet. Diese bekannten Knochenplatten, bei denen die Mittel zum Festlegen der Knochenschraube als umlaufende Lippe gebildet sind, erlauben eine Festlegung der Knochenschrauben in einem Winkelbereich zwischen 0° und 15°.

Als nachteilig an diesem Stand der Technik hat sich neben diesem eingeschränkten Winkelbereich der große Kraftaufwand herausgestellt, der zum Umformen der umlaufenden Lippe beim Eindrehen der Knochenschraube aufgebracht werden muss.

Die Erfindung hat es sich daher zur Aufgabe gestellt, eine gattungsgemäße Knochenplatte zu schaffen, die ein leichteres Eindrehen der Knochenschraube bei einem größerem Winkelbereich erlaubt.

Erfindungsgemäß wird dies dadurch erreicht, dass die Mittel zum Festlegen der Knochenschraube wenigstens zwei, vorzugsweise vier, in einer zur Knochenplatte im Wesentlichen parallelen Ebene angeordnete Vorsprünge umfassen, wobei eine besonders stabile Fixierung der Knochenschraube in ihrer Winkelstellung zur Knochenplatte dann erreicht werden kann, wenn die Mittel zum Festlegen der Knochenschraube sechs oder acht Vorsprünge umfassen.

Dadurch, dass die Mittel zum Festlegen der Knochenschraube von voneinander beabstandeten Vorsprüngen gebildet werden, die gemäß einem weiteren Ausführungsbeispiel der Erfindung über den Umfang der Durchtrittsöffnung im Wesentlichen gleichmäßig verteilt angeordnet und im Querschnitt dreieckig und/oder trapezförmig ausgebildet sind, kann die Knochenschraube in einem Winkel zwischen 0° und 40°- bezogen auf die Längsachse der Durchtrittsöffnung - angesetzt und eingeschraubt werden.

Ein weiterer Vorteil, der sich durch die beabstandete Anordnung der Vorsprünge ergibt, besteht in dem Umstand, dass beim Eindrehen der Knochenschraube, die man auch als Gewindedränger bezeichnen könnte, lediglich die zur Auslenkrichtung der Knochenschraube im Wesentlichen parallel verlaufenden Vorsprünge einer starken Umformung unterworfen sind, während hingegen die zur Auslenkrichtung der Knochenschraube normal angeordneten Vorsprünge kaum verformt werden, sodass gegenüber den bekannten Vorrichtungen ein wesentlich geringerer Kraftaufwand notwendig ist, um die Knochenschraube in einem bestimmten Winkel festzulegen. Dadurch ist es weiters möglich, die Mittel zum Festlegen der Schraube aus zumindest annähernd gleich hartem Material wie die Knochenschraube herzustellen. Beim Stand der Technik war dies bedingt durch die große Umformungsfläche und dem damit verbundenen Kraftaufwand nicht möglich, weshalb die umformbare Lippe aus weicherem und damit verschleißanfälligerem Material hergestellt werden musste.

Eine bevorzugte Ausführungsvariante der Erfindung sieht weiters vor, dass die wenigstens eine Durchtrittsöffnung zumindest oberhalb der Mittel zum Festlegen der Knochenschraube zylinderförmig, vorzugsweise kreiszylinderförmig, ausgebildet ist, wobei es sich als günstig erwiesen hat, wenn die Vorsprünge kreisringsegmentförmig ausgebildet und über den Umfang der Durchtrittsöffnung im Wesentlichen gleichmäßig verteilt angeordnet sind. Bei einer derartigen Ausbildung können die von der Umformung stark betroffenen Vorsprünge, dadurch dass sie von den von der Umformung weniger stark betroffenen Vorsprüngen beabstandet sind, leichter verbogen werden, sodass der Kraftschluss und/oder Stoffschluss zwischen den Vorsprüngen und der Knochenschraube auf kürzerem Weg erreicht wird als bei den bisher bekannten Vorrichtungen.

Eine herstellungstechnisch besonders einfache Konstruktion ergibt sich, wenn gemäß einem weiteren Ausführungsbeispiel der Erfindung die Mittel zum Festlegen der Knochenschraube am unteren Ende der Durchtrittsöffnung, vorzugsweise mit der Knochenplatte plan abschließend, angeordnet sind.

Gemäß einem weiteren Aspekt der Erfindung soll die neuartige Knochenplatte als Teil eines knochenchirurgischen Fixiersystems zusammen mit mehreren Knochenschrauben verwendet werden. Dabei hat es sich für einen besonders sicheren und winkelstabilen Sitz der Knochenschraube in der Knochenplatte als vorteilhaft erwiesen, wenn der Schraubenkopf wenigstens einer Knochenschraube zumindest bereichsweise sphärisch ausgebildet ist und die sphärischen Bereiche zumindest teilweise mit einem Gewinde versehen sind.

Gemäß einem weiteren Ausführungsbeispiel der Erfindung bilden dabei die Mittel zum Festlegen der Schraube im Gebrauchszustand des knochenchirurgischen Fixiersystems durch zumindest teilweise Umformung eine Gewindeverbindung mit dem an den sphärischen Bereichen des Schraubenkopfes ausgebildeten Gewinde, sodass die Knochenschraube unverrückbar in der Durchtrittsöffnung der Knochenplatte fixiert werden kann.

Weitere Einzelheiten der Erfindung und der durch sie erzielten Vorteile ergeben sich aus der nachstehenden Beschreibung der in der Zeichnung dargestellten Ausführungsbeispiele der Erfindung. Darin zeigt:
- Fig. 1: schematisch eine Prinzipskizze einer Knochenplatte mit eingesetzter Knochenschraube,
- Fig. 2: schematisch in Draufsicht einen Teilausschnitt einer erfindungsgemäßen Knochenplatte,
- Fig. 3a und 3b: eine Draufsicht auf eine erfindungsgemäße Knochenplatte und einen Querschnitt durch einen Teilabschnitt der Knochenplatte entlang B-B',
- Fig.4: ein Ausführungsbeispiel einer Knochenschraube zur Verwendung in einem erfindungsgemäßen knochenchirurgischen Fixiersystem und
- Fig. 5: ein weiteres Ausführungsbeispiel der Erfindung.

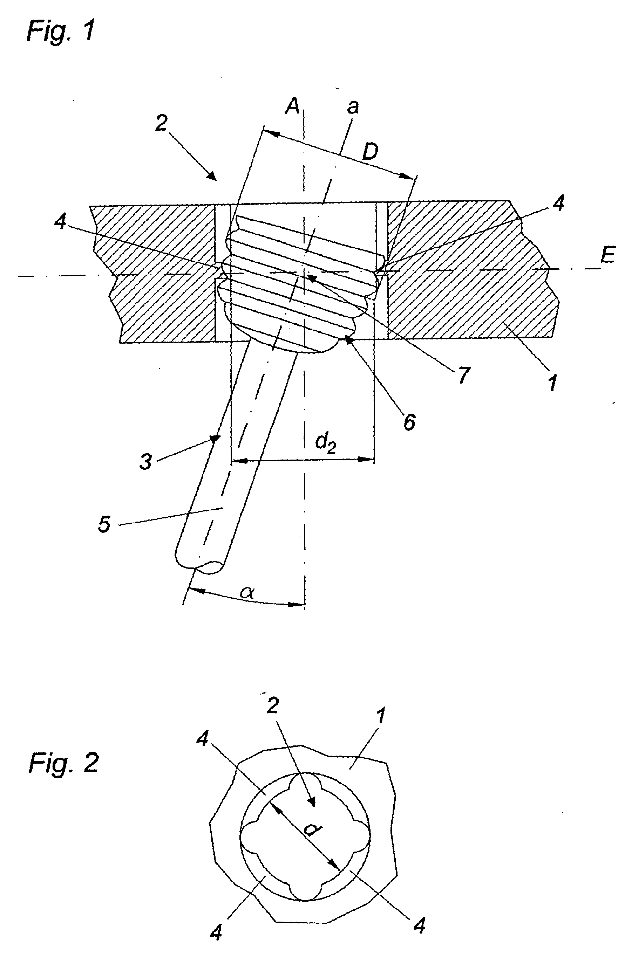

Die in Fig. 1 dargestellte Knochenplatte 1 weist eine zylindrische Durchtrittsöffnung 2 zur Aufnahme einer Knochenschraube 3 auf. In der zylindrischen Durchtrittsöffnung 2 sind Mittel 4 zum winkelstabilen Festlegen einer Knochenschraube 3 angeordnet, wobei die Mittel 4 zum Festlegen der Knochenschraube 3 in einer zur Ebene der Knochenplatte 1 im Wesentlichen parallelen Ebene E angeordnet sind. Die Knochenschraube 3, die einen Schraubenkopf 6 und einen Schaft 5 umfasst, kann in der Durchtrittsöffnung 2 der Knochenplatte 1 unter einem Winkel α festgelegt werden. Der Winkel α, den die Längsachse a der Knochenschraube 3 mit der Längsachse A der Durchtrittsöffnung 2 einschließt, kann dabei zwischen 0° und etwa 40° liegen.

Die winkelstabile Festlegung der Knochenschraube 3 in der Knochenplatte 1 erfolgt dabei durch eine Gewindeverbindung zwischen den zumindest teilweise umgeformten Mitteln 4 und dem am halbkugelförmig ausgebildeten Schraubenkopf 6 angeordneten Gewinde 7. Anders ausgedrückt bedeutet das, dass die umgeformten Mittel 4 zum Festlegen der Knochenschraube 3 bei eingesetzter Knochenschraube 3 eine Gewindeverbindung mit dem Gewinde 7 eingehen und somit gleichzeitig eine Sitzfläche für den Schraubenkopf 6 bilden.

Der Außendurchmesser D des Schraubenkopfes 6 entspricht in etwa dem lichten Durchmesser der Durchtrittsöffnung 2. Hingegen ist der lichte Durchmesser d der kreisringsegmentförmig angeordneten Mittel 4 zum Festlegen der Knochenschraube (Fig. 2) wesentlich kleiner als der Durchmesser der Durchtrittsöffnung 2. Beim Eindrehen der Knochenschraube in die Durchtrittsöffnung weitet sich der lichte Durchmesser d durch Umformung der Mittel 4 zum Festlegen der Knochenschraube auf, sodass der lichte Durchmesser d₂ bei eingesetzter Knochenschraube 3 (siehe Fig. 1) nur mehr geringfügig kleiner ist als der Durchmesser der Durchtrittsöffnung 2.

Versuche des Anmelders haben ergeben, dass die Knochenschraube 3 umso fester in der Durchtrittsöffnung 2 sitzt je näher sich der Mittelpunkt des zumindest teilweise sphärisch ausgebildeten Schraubenkopfes 6 der Ebene E, in der die Mittel 4 zum Festlegen der Knochenschraube angeordnet sind, nähert. Anders ausgedrückt, kann ein fester Sitz der Knochenschraube 3 in der Durchtrittsöffnung 2 dann erreicht werden, wenn der Lichtdurchmesser d₂ nur mehr geringfügig kleiner ist als der Durchmesser der Durchtrittsöffnung 2.

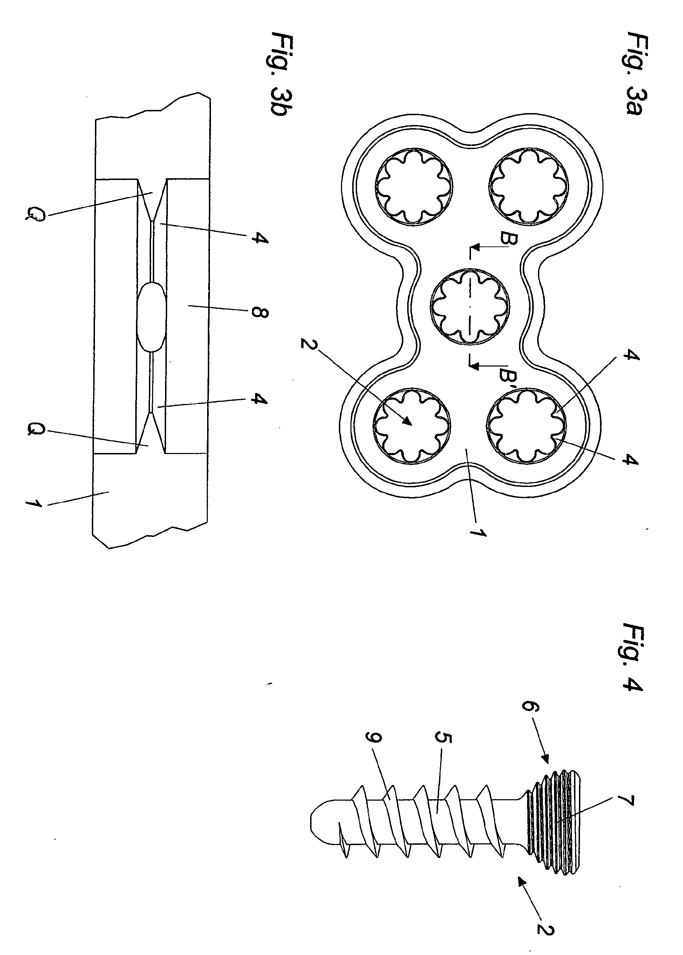

Fig. 3a zeigt ein weiteres Ausführungsbeispiel einer erfindungsgemäßen Knochenplatte 1 mit mehreren Durchtrittsöffnungen 2, in denen die Mittel 4 zum Festlegen einer Knochenschraube 3 von jeweils acht Vorsprüngen, die über den Umfang der Durchtrittsöffnung 2 gleichmäßig verteilt sind, gebildet werden.

Fig. 3b zeigt einen Querschnitt durch einen Teilabschnitt der Knochenplatte 1 aus Fig. 3a entlang der Schnittlinie B-B'. Daraus ist ersichtlich, dass die Mittel 4 zum Festlegen der Knochenschraube 3 in etwa in der Mitte der Knochenplatte 1 an der Wandung 8 der Durchtrittsöffnung 2 angeordnet sind und einen dreieck- bzw. trapezförmigen Querschnitt Q aufweisen, das heißt, die Mittel 4 verjüngen sich zum Mittelpunkt der Durchtrittsöffnung 2 hin.

Fig. 4 zeigt ein bevorzugtes Ausbildungsbeispiel einer Knochenschraube 3 zur Verwendung in einem erfindungsgemäßen knochenchirurgischen Fixiersystem. Die Knochenschraube 3 weist einen Schraubenkopf 6 auf, der halbkugelförmig ausgebildet ist und ein Gewinde 7 aufweist. Unterhalb des Schraubenkopfes 6 verläuft der Schaft 5 der Knochenschraube 3, wobei am Schaft 5 ein Gewinde 9 zum Festlegen der Knochenschraube 3 im Knochen angeordnet ist. Das Gewinde 7 am Schraubenkopf 6 kann, ebenso wie das Gewinde 9 am Schraubenschaft 5, selbstschneidend ausgebildet sein.

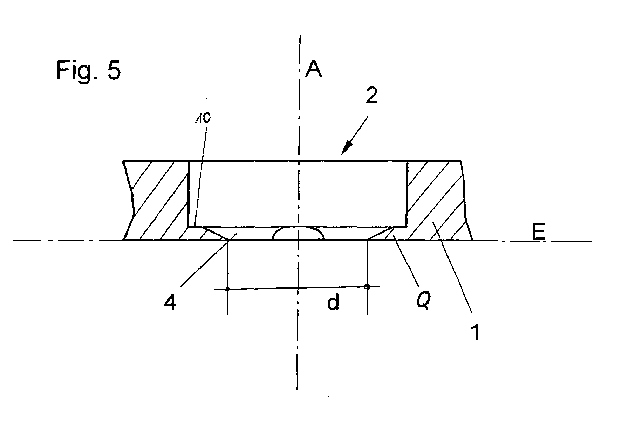

Fig. 5 zeigt einen Querschnitt durch ein weiteres Ausführungsbeispiel einer erfindungsgemäßen Knochenplatte 1, bei dem die Mittel 4 zum Festlegen der Knochenschraube 3 im unteren Endbereich der Durchtrittsöffnung 2 angeordnet sind und im Querschnitt Q im Wesentlichen in Form eines rechtwinkligen Dreiecks ausgebildet sind. Der flanschartige Vorsprung 10 ist durch die Art der Herstellung der Durchtrittsöffnung 2 und der Mittel 4 bedingt, da bei der Herstellung zuerst ein Führungsloch entlang der Mittellängsachse A der Durchtrittsöffnung 2 gebohrt wird. In dieses Führungsloch wird dann ein Fräser angesetzt, mit dem die Durchtrittsöffnung 2 von oben aus der Knochenplatte gefräst wird und zwar so weit, bis die Knochenplatte 1 im Bereich der Durchtrittsöffnung 2 nur mehr die Stärke der Mittel 4 zum Festlegen der Knochenschraube aufweist. Abschließend wird dann der verbleibende scheibenförmige Kreisring wiederum von oben mit einem Bohrer, dessen Durchmesser etwas kleiner ist als der Durchmesser der Durchtrittsöffnung, abgeschrägt. Die Ausbildung der einzelnen Segmente der Mittel 4 kann dann zum Schluss erfolgen.

Im Übrigen sei noch angeführt, dass die dargestellten Ausführungsbeispiele lediglich Prinzipskizzen sind und keinen Schluss auf die tatsächlichen Größenverhältnisse zulassen. Bei einem Ausführungsbeispiel der Erfindung ist die Knochenplatte beispielsweise lediglich 1,5 mm stark, wobei die Mittel 4 eine Stärke von lediglich 0,2 mm aufweisen.

Die dargestellten Ausführungsbeispiele von Knochenplatten und Knochenschrauben sind selbstverständlich nicht in einschränkendem Sinne zu verstehen, sondern eben nur einzelne Beispiele von zahlreichen Möglichkeiten, den Erfindungsgedanken einer Knochenplatte bzw. eines knochenchirurgischem Fixiersystems zu verwirklichen. Eine Grundidee der Erfindung liegt jedenfalls in der Anordnung voneinander beabstandeter Mittel zum Festlegen einer Knochenschraube in einer Durchtrittsöffnung einer Knochenplatte. Eine weitere Grundidee der Erfindung besteht darin, die Durchtrittsöffnung in der Knochenplatte oberhalb der Mittel zum Festlegen der Schraube zylindrisch auszubilden, sodass die Mittel zum Festlegen der Schraube im eingesetzten Zustand der Schraube gleichzeitig als Sitzfläche für die Schraube dienen.

## Patentansprüche

1. Knochenplatte mit wenigstens einer Durchtrittsöffnung für eine Knochenschraube, wobei in der Durchtrittsöffnung verformbare Mittel zum winkelstabilen Festlegen einer Knochenschraube unter verschiedenen Winkeln angeordnet sind, **dadurch gekennzeichnet, dass** die Mittel (4) zum Festlegen der Knochenschraube (3) wenigstens zwei, vorzugsweise vier, in einer zur Knochenplatte (1) im Wesentlichen parallelen Ebene (E) angeordnete Vorsprünge umfassen.

2. Knochenschraube nach Anspruch 1, **dadurch gekennzeichnet, dass** die Mittel (4) zum Festlegen der Knochenschraube (3) sechs oder acht Vorsprünge umfassen.

3. Knochenplatte nach einem der Ansprüche 1 oder 2, **dadurch gekennzeichnet, dass** die Vorsprünge über den Umfang der Durchtrittsöffnung (2) im Wesentlichen gleichmäßig verteilt angeordnet sind.

4. Knochenplatte nach einem der Ansprüche 1 bis 3, **dadurch gekennzeichnet, dass** die Vorsprünge im Querschnitt (Q) dreieckig und/oder trapezförmig ausgebildet sind.

5. Knochenplatte mit wenigstens einer Durchtrittsöffnung für eine Knochenschraube, wobei in der Durchtrittsöffnung verformbare Mittel zum winkelstabilen Festlegen einer Knochenschraube unter verschiedenen Winkeln abgeordnet sind, insbesondere nach einem der Ansprüche 1 bis 4, **dadurch gekennzeichnet, dass** die wenigstens eine Durchtrittsöffnung (2) zumindest oberhalb der Mittel (4) zum Festlegen der Knochenschraube (3) zylinderförmig, vorzugsweise kreiszylinderförmig, ausgebildet ist.

6. Knochenplatte nach Anspruch 5, **dadurch gekennzeichnet, dass** die Vorsprünge kreisringsegmentförmig ausgebildet und über den Umfang der Durchtrittsöffnung (2) im Wesentlichen gleichmäßig verteilt angeordnet sind.

7. Knochenplatte nach einem der Ansprüche 1 bis 6, **dadurch gekennzeichnet, dass** die Mittel (4) zum Festlegen der Knochenschraube (3) am unteren Ende der Durchtrittsöffnung (2), vorzugsweise mit der Knochenplatte (1) plan abschließend, angeordnet sind.

8. Verwendung einer Knochenplatte nach einem der Ansprüche 1 bis 7 als Teil eines knochenchirurgischen Fixiersystems zusammen mit mehreren Knochenschrauben.

9. Knochenchirurgisches Fixiersystem nach Anspruch 8, **dadurch gekennzeichnet, dass** der Schraubenkopf (6) wenigstens einer Knochenschraube (3) zumindest bereichsweise sphärisch ausgebildet ist und die sphärischen Bereiche zumindest teilweise mit einem Gewinde (7) versehen sind.

10. Knochenchirurgisches Fixiersystem nach Anspruch 9, **dadurch gekennzeichnet, dass** die Mittel (4) zum Festlegen der Knochenschraube (3) im Gebrauchszustand des knochenchirurgischen Fixiersystems durch zumindest teilweise Umformung eine Gewindeverbindung mit dem an den sphärischen Bereichen des Schraubenkopfes (6) ausgebildeten Gewinde (7) bilden.
